# EUROPEAN PATENT SPECIFICATION

(11) **EP 2 566 449 B1**
(45) Date of publication and mention of the grant of the patent: **08.10.2014**
(21) Application number: 11723765.1
(22) Date of filing: 02.05.2011
(51) Int. Cl.: A61K 9/00, A61K 9/16, A61K 9/20, A61K 31/545

(54) **PHARMACEUTICAL COMPOSITIONS COMPRISING CEFTIBUTEN**
PHARMAZEUTISCHE ZUSAMMENSETZUNGEN MIT CEFTIBUTEN
COMPOSITIONS PHARMACEUTIQUES COMPRENANT DU CEFTIBUTÈNE

(30) Priority: 04.05.2010 TR 201003546
(43) Date of publication of application: 13.03.2013
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000129
(87) International publication number: WO 2011/139253

(56) References cited:
- WO-A1-01/62231
- WO-A1-93/21923
- US-A1- 2006 099 253
- DATABASE WPI Week 200957 Thomson Scientific, London, GB; AN 2009-L39291 XP002672584, -& CN 101 468 021 A (BEIJING HOPE HUGE PHARM SCI CO LTD) 1 July 2009 (2009-07-01)
- DATABASE WPI Week 200956 Thomson Scientific, London, GB; AN 2009-L39295 XP002672585, -& CN 101 468 017 A (BEIJING HOPE HUGE PHARM SCI CO LTD) 1 July 2009 (2009-07-01)
- ISP: "Plasdone povidones: Product overview", PHARMACEUTICALS ISP, April 2010 (2010-04), pages 1-4, XP002672586,

## Description

The present invention relates to effervescent formulations comprising ceftibuten and/or its pharmaceutically acceptable salts, hydrates, solvates, esters, amorphous and crystal forms and/or a combination thereof.

### Background of the Invention:

Ceftibuten, which has the chemical name (+)-(6*R*,7*R*)-7-([(*Z*)-2-(2-amino-4-thiazolyl)-4-*carboxycrotonamido*] -8-oxo-5-thia-1-azabicyclo[4.2.0]oct-2-ene-2-carboxylic acid, was first disclosed in the patent numbered US 4634697. It was indicated in the patents numbered US 4933443 and US 4812561 that ceftibuten is stable in dihydrate and trihydrate forms. Moreover, it was indicated in said patents that dihydrate and trihydrate forms of ceftibuten can be filled into hard gelatin capsules for administration by the oral route.

However, the use of such oral dosage forms is not preferred since they become quite large in size and this made the use of this dosage form inconvenient in patients with dysphagia, particularly in pediatric and geriatric patients.

The suspension forms have been alternatively developed to overcome said problems and dry powder formulations comprising ceftibuten which are suitable for use in suspension form were disclosed in the patent numbered EP0642344. However, they are not preferred since the potential of high and/or uncontrolled dose intake and they are not physically and chemically stable. Alternatively, the use of reliable and user-friendly effervescent forms is suggested.

The document WO0162231 discloses in example 14 an intermediate formulation comprising ceftibuten, PEG 4000 and povidone. The document CN101468021A discloses enteric-coated ceftibuten tablets comprising sodium carboxymethylstarch, lactose, povidone and talc as core excipients. These documents do not disclose an effervescent formulation and the ratio of ceftibuten to povidone in the range from 7:1 to 4:1.

The physical properties of the final product in the effervescent form such as hardness and dissolution time can change according to the formulations developed. These changes in the physical properties of the effervescent formulations are of great importance on the preparation and usage of the final product. In cases where the effervescent formulations comprising ceftibuten are compressed into effervescent tablet, dissolution time has an important effect on the disintegration of these effervescent tablets in water.

In cases where the effervescent formulations comprising ceftibuten are compressed into effervescent tablet, increase in the tablet hardness leads to the increase in dissolution time of the ceftibuten formulations in water. This causes increase of time period it takes to get the drug ready for use and thus leads to an inefficient delivery of the drug.

As is seen, new formulations comprising ceftibuten are required to be developed in order to provide new formulations which are quickly dispersed in water during use and convenient to administer effectively.

The inventors have surprisingly found that said problems in the prior art can be solved by the effervescent formulations prepared according to the subject of the present invention.

### Description of the Invention:

The subject of the present invention relates to the effervescent formulations comprising ceftibuten and/or its pharmaceutically acceptable salts, hydrates, solvates, esters, amorphous and crystal forms and/or a combination thereof and the procedures for their preparation. Surprisingly, it has been found that the effervescent formulations comprising ceftibuten in which povidone having an average particle size in the range of 60-150 µm is used and the ratio of ceftibuten to said binder is in the range of 7:1 to 4:1, has an optimum hardness and disintegrate quickly in water, and provide an effective administration during use.

According to this, the present invention relates to effervescent formulations comprising ceftibuten in which povidone having an average particle size in the range of 60-150µm is used, the ratio of ceftibuten to said binder is in the range of 7:1 to 4:1 and the amount of povidone is in the range of 1-5% of the total unit dose weight.

Based on the studies of the inventors, in order to obtain ceftibuten effervescent tablets which have desirable hardness and are convenient for the effective administration, it is found that the optimum hardness of the effervescent tablets is in the range of 6-8 kP and the optimum dissolution time is maximum 5 minutes.

According to this, another aspect of the present invention is that the ceftibuten effervescent forms, in which povidone binder having an average particle size of 60-150µm is used and the ratio of ceftibuten to povidone is in the range of 7:1 to 4:1, have a hardness value in the range of 6-8 kP and dissolve in water within 5 minutes.

The term "effervescent formulations" present in the text comprises effervescent tablets, effervescent granules and effervescent powders.

Ceftibuten can be present in the form of its solvates, monohydrates, dihydrates, trihydrates, esters, enantiomers, racemates, organic salts, inorganic salts, polymorphs, crystal and amorphous forms or in free form and/or a combination thereof in the effervescent formulations. Preferably, ceftibuten dihydrate and/or trihydrate is used in scope of the present invention.

According to these, another aspect of the present invention is effervescent formulations comprising ceftibuten dihydrate and/or trihydrate in which povidone as a binder having an average particle size in the range of 60-150µm is used and the ratio of ceftibuten to povidone is in the range of 7:1 to 4:1.

The inventors have found that using povidone in the range of 1-5%, with respect to total weight of the unit dose has a positive effect on the disintegration of the effervescent formulations comprising ceftibuten in water.

Another aspect of the present invention is effervescent formulations comprising ceftibuten as the active agent, at least one non-cellulose-based binder and pharmaceutically acceptable excipients.

Various excipients selected from, but not limited to, a group comprising lubricants, disintegrants, diluent, taste regulating agents, effervescent acid and effervescent base can be used in the effervescent formulations.

The lubricant that can be used in the effervescent formulations pertaining to the present invention can be selected from, but not limited to, a group comprising calcium stearate, magnesium stearate, polyethylene glycol, PEG 6000, polyvinyl alcohol, potassium benzoate, sodium benzoate. Preferably, PEG 6000 is used as the lubricant in scope of the present invention.

The disintegrant that can be used in effervescent formulations pertaining to the present invention can be selected from, but not limited to, a group comprising carboxymethyl cellulose calcium, carboxymethyl cellulose sodium, microcrystalline cellulose, silicon dioxide, croscarmellose sodium, crospovidone, hydroxypropyl cellulose, methyl cellulose, povidone, magnesium aluminum silicate and starch or combinations thereof.

The diluent that can be used in effervescent formulations pertaining to the present invention can be selected from, but not limited to, a group comprising calcium carbonate, calcium sulphate, dibasic calcium phosphate, tribasic calcium phosphate, calcium phosphate, calcium sulphate, microcrystalline cellulose, lactose, magnesium carbonate, magnesium oxide, maltodextrin, maltose, mannitol, sodium chloride, sorbitol, starch and xylitol or combinations thereof.

The taste regulating agent that can be used in effervescent formulations pertaining to the present invention can be selected from, but not limited to, a group comprising acesulfame, aspartame, dextrose, fructose, glucose, lactitol, maltitol, maltose, sorbitol, saccharine, saccharine sodium, saccharose, sodium cyclamate, sucralose, sodium chloride, potassium chloride, sucrose, xylitol or combinations thereof. Preferably, sucrose, aspartame, sodium chloride or a combination thereof is used in the formulations according to the present invention. Preferably, saccharose, aspartame, sodium chloride or a combination thereof is used as the taste regulating agent in the effervescent formulations pertaining to the present invention.

Ceftibuten is a molecule having a bitter taste. Therefore, this problem should be taken into consideration when developing effervescent formulations comprising this molecule.

Therefore, the inventors have found that the bitter taste of the effervescent forms comprising ceftibuten is eliminated and a pleasant taste is achieved in the case that the ratio of the taste regulating agent is used in an amount of 1-8%, preferably 2-6%, most preferably 3-5% with respect to the total unit dose weight.

According to this, another aspect of the present invention is effervescent formulations in which the ratio of the taste regulating agent is 1-8%, preferably 2-6%, most preferably 3-5% with respect to total unit dose weight.

According to another aspect, the ratio of ceftibuten to the taste regulating agent is in the range of 1:1 to 4:1, preferably 2:1 to 3:1 in the effervescent formulations which are the subject of the present invention.

Effervescent acid that is used in effervescent formulations pertaining to the present invention can be selected from organic acids such as citric acid, tartaric acid, malic acid, fumaric acid, ascorbic acid, adipic acid.

Effervescent base that is used in effervescent formulations pertaining to the present invention can be selected from basic agents such as sodium hydrogen carbonate, sodium carbonate, potassium carbonate, potassium bicarbonate, sodium glycine carbonate, lysine carbonate, arginine carbonate and calcium carbonate.

The effervescent formulations pertaining to the present invention may comprise ceftibuten or pharmaceutically acceptable solvates, monohydrate form, dihydrate form, trihydrate form, enantiomers, racemates, organic salts, inorganic salts, polymorphs, crystal and amorphous forms of 5-50% of the total weight of the unit dose.

The effervescent formulation of the present invention can comprise 5-50% ceftibuten; 1-5% binder; 0.1-8% lubricant; 0-15% disintegrant and/or disintegrants; 0-55% diluent; 1-8% taste regulating agent; 0.5-5% flavoring agents and 10-45% effervescent acid and 20-75% effervescent base with respect to the total amount of unit dose.

In another aspect, the present invention relates to the processes which can be used for the preparation of single dose effervescent formulations comprising pharmaceutically acceptable excipients in addition to ceftibuten as the active agent.

The pharmaceutical compositions of the present invention can be prepared through a process which is comprised of dry blending or dry granulation or wet granulation of the components or a combination thereof and generally known standard techniques and manufacture procedures in technology.

In another aspect, the present invention relates to the preparation of a medicament comprising ceftibuten so as to be used in the treatment of infections caused by gram positive and gram negative bacteria.

According to another aspect of the present invention, the pharmaceutical composition prepared according to the present invention is used in thetreatment of upper respiratory infections such as ear, nose, throat, otitis media, sinusitis, tonsillitis, pharyngitis; lower respiratory tract infections such as pyelonephritis, cystitis and urethritis; skin or soft tissue infections such as froncle, pyoderma, impetigo; in the treatment and prophylaxis of gonorrhea and lyme diseases.

The effervescent formulations pertaining to the present invention can be prepared as described below, but not limited to these examples.

### EXAMPLE 1: Formulation for the preparation of effervescent granules

| | % of amount present in unit dose |
|---|---|
| Ceftibuten dihydrate | 13.0% |
| Effervescent acid | 28.0% |
| Effervescent base | 51.0% |
| Povidone | 2.0% |
| Taste regulating agent | 3.5% |
| Lubricant | 1.5% |
| Aroma | 1.0% |

The pharmaceutical composition pertaining to the present invention is prepared through granulation of effervescent couple and taste regulating agent with a granulation solution comprising povidone and a suitable solvent; drying the obtained granules; and then mixing them with ceftibuten, lubricant and aroma.

### EXAMPLE 2: Formulation for the preparation of effervescent tablets

| | % of amount present in unit dose |
|---|---|
| *Ceftibuten dihydrate | 10.0% |
| Effervescent acid | 33.0% |
| Effervescent base | 49.0% |
| *Povidone | 2.5% |
| Taste regulating agent | 3.0% |
| Lubricant | 1.0% |
| Aroma | 1.5% |

| | |
|---|---|
| * Ceftibuten Dihydrate : povidone ratio is 4:1. | |

The pharmaceutical composition pertaining to the present invention is prepared through granulation of effervescent couple and taste regulating agent with a granulation solution comprising povidone having a particle size of 100 µm and a suitable solvent; drying the obtained granules; then mixing them with ceftibuten, lubricant and aroma; and finally compressing them into tablets.

### COMPARATIVE EXAMPLE 1: Formulation for the preparation of effervescent tablets

| | % of amount present in unit dose |
|---|---|
| *Ceftibuten dihydrate | 7.5% |
| Effervescent acid | 33.5% |
| Effervescent base | 51.0% |
| *Povidone | 2.5% |
| Taste regulating agent | 3.0% |
| Lubricant | 1.0% |
| Aroma | 1.5% |

| | |
|---|---|
| * Ceftibuten Dihydrate : povidone ratio is 3:1 | |

The pharmaceutical composition pertaining to the present invention is prepared through granulation of effervescent couple and taste regulating agent with a granulation solution comprising povidone having a particle size of 200 µm and a suitable solvent; drying the obtained granules; then mixing them with ceftibuten, lubricant and aroma; and finally compressing them into tablets.

The inventors have studied on the values of the hardness and dissolution time of the effervescent compositions compressed in the effervescent tablet which are illustrated in Example 2 and Comparative example 1. According to the studies based on the comparison of their hardness and dissolution time values, the results represented in Tables 1 are observed.

**Table1. Comparative Data of Hardness and Dissolution Rate of Ceftibuten Compositions in Effervescent Tablet Form**

| Sample | Average Particle Size of Povidone (µm) | Ceftibuten : Povidone Ratio | Hardness (kP) | Dissolution Time (') minute (") second |
|---|---|---|---|---|
| Composition of Example 2 | 100 | 5:1 | 7 | 2' 38" |
| Composition of Comparative Example 1 | 200 | 3:1 | 10 | 5' 75" |

Table 1 indicates the comparative data of hardness and dissolution time of the compositions for each example. The results clearly show that hardness of the composition of example 1 is in the range of 6-8 kP and thus the dissolution time of this example is below 5 minutes. Therefore, it can be concluded that the effervescent tablet of the present invention in which average particle size of povidone is in the range of 60-150µm and the ratio of ceftibuten to povidone is in the range of 7:1 to 4:1 is much preferred via its optimum hardness and dissolution time.

## Claims

1. An effervescent pharmaceutical composition comprising ceftibuten, **characterized in that**;
• povidone as binder having an average particle size in the range of 60-150µm is used,
• the ratio of ceftibuten to povidone is in the range of 7:1 to 4:1 and
• the amount of povidone is in the range of 1-5% of the total unit dose weight.

2. The effervescent pharmaceutical composition according to claim 1, wherein ceftibuten can be in the form of its solvates, monohydrates, dihydrates, trihydrates, esters, enantiomers, racemates, organic salts, inorganic salts, polymorphs, in crystal and amorphous form or in free form and/or combination of thereof.

3. The effervescent pharmaceutical composition according to claim 2, wherein ceftibuten is in the form of dihydrate and/or trihydrate.

4. The effervescent pharmaceutical composition according to claims 1-3, wherein said composition comprises lubricants, disintegrants, diluents, taste regulating agents, effervescent acid and effervescent base as pharmaceutically acceptable excipients.

5. The effervescent pharmaceutical composition according to claim 4, wherein the taste regulating agent is selected from a group comprising acesulfame, aspartame, dextrose, fructose, glucose, lactitol, maltitol, sorbitol, saccharine, saccharine sodium, saccharose, sodium cyclamate, sucralose, sodium chloride, potassium chloride, sucrose, xylitol or combinations thereof.

6. The effervescent pharmaceutical composition according to claim 1-5, wherein 1-8% taste regulating agent is used with respect to the unit dose weight.

7. The effervescent pharmaceutical composition according to claim 6, wherein the ratio of ceftibuten to the taste regulating agent is in the range of 1:1 to 4:1.

8. The effervescent pharmaceutical composition according to claim 7, wherein the ratio of ceftibuten to the taste regulating agent is 3:1.

9. The effervescent pharmaceutical composition according to claim 4, wherein the effervescent acid is selected from organic acids such as citric acid, tartaric acid, malic acid, fumaric acid, ascorbic acid, adipic acid.

10. The effervescent pharmaceutical composition according to claim 4, wherein the effervescent base is selected from basic agents such as sodium hydrogen carbonate, sodium carbonate, potassium carbonate, potassium bicarbonate, sodium glycine carbonate, lysine carbonate, arginine carbonate and calcium carbonate.

11. The effervescent pharmaceutical composition according to claims 1-10, wherein said composition may comprise 5-50% ceftibuten or its pharmaceutically acceptable solvates, monohydrate form, dihydrate form, trihydrate form, enantiomers, racemates, organic bases, inorganic bases, polymorphs, crystal and amorphous forms with respect to the total weight of the unit dose.

12. The effervescent pharmaceutical composition according to claims 1-11, wherein said composition comprises 5-50% ceftibuten; 1-5% povidone as binder; 0.1-8% lubricant; 0-15% disintegrant and/or disintegrants; 0-55% diluent; 1-8% taste regulating agent; 0.5-5% flavoring agents and 10-45% effervescent acid and 20-75% effervescent base with respect to the total amount of unit dose.

13. The effervescent pharmaceutical composition according to claims 1-12, wherein said composition is prepared through a process comprised of dry blending or dry granulation or wet granulation of the components or a combination thereof.

## Patentansprüche

1. Eine pharmazeutische Brausezusammensetzung, die Ceftibuten enthält und **dadurch gekennzeichnet, dass**;
• povidon mit der durchschnittlichen Teilchengröße zwischen 60-150µm wird als Bindemittel verwendet,
• das Verhältnis von Ceftibuten zu dem Povidon ist im Bereich von 7:1 bis 4:1 und
• die Menge von Povidon ist im Bereich von 1-5% vom Gewicht der Gesamtdosiseinheit.

2. Die pharmazeutische Brausezusammensetzung gemäß Anspruch 1 und **dadurch gekennzeichnet, dass** Ceftibuten in Form seiner Solvate, Monohydrate, Dihydrate, Trihydrate, Ester, Enantiomere, Racemate, organischen Salzen, anorganischen Salzen, Polymorphe, in Kristall und amorpher Form oder in freier Form und/oder Kombination davon sein kann.

3. Die pharmazeutische Brausezusammensetzung gemäß Anspruch 2 und **dadurch gekennzeichnet, dass** Ceftibutenin Form von Dihydrat und/oder Trihydrat ist.

4. Die pharmazeutische Brausezusammensetzung gemäß Ansprüche 1-3 und **dadurch gekennzeichnet**, dassdie genannte Zusammensetzung Gleitmittel, Sprengmittel, Verdünnungsmittel, Geschmacksregulierungsmittel, Brausesäure und Brausebasis als Hilfsstoff, das als pharmazeutisch angenommen werden kann, enthält.

5. Die pharmazeutische Brausezusammensetzung gemäß Anspruch 4 und **dadurch gekennzeichnet, dass** Geschmackregulierendes Mittel aus einer Gruppe ausgewähltwird, die Acesulfam, Aspartam, Dextrose, Fructose, Glucose, Lactitol, Maltitol, Sorbitol, Saccharin, Saccharin-Natrium, Saccharose, Natriumcyclamat, Sucralose, Natriumchlorid, Kaliumchlorid, Saccharose, Xylitol oder Kombinationen davon enthält.

6. Die pharmazeutische Brausezusammensetzung gemäß Ansprüche 1-5 und **dadurch gekennzeichnet, dass** Geschmackregulierendes Mittel von 1-8% in Bezug auf das Gewicht der Dosiseinheit verwendet wird.

7. Die pharmazeutische Brausezusammensetzung gemäß Anspruch 6 und **dadurch gekennzeichnet, dass** das Verhältnis von Ceftibuten im Bereich zwischen 1:1 und 4:1 zu dem Geschmackregulierendes Mittel ist.

8. Die pharmazeutische Brausezusammensetzung gemäß Anspruch 7 und **dadurch gekennzeichnet, dass** das Verhältnis von Ceftibuten im Bereich von 3:1 zu dem Geschmackregulierendes Mittel ist.

9. Die pharmazeutische Brausezusammensetzung gemäß Anspruch 4 und **dadurch gekennzeichnet, dass** Brausesäure aus organischen Säuren, wie Zitronensäure, Weinsäure, Äpfelsäure, Fumarsäure, Ascorbinsäure, Adipinsäure ausgewählt wird.

10. Die pharmazeutische Brausezusammensetzung gemäß Anspruch 4 und **dadurch gekennzeichnet, dass** Brausebasis aus basischen Mitteln, wie Natriumhydrogencarbonat, Natriumcarbonat, Kaliumcarbonat, Kaliumbicarbonat, Natrium-Glycin-Carbonat, Lysin- Carbonat, Arginin- und Calciumcarbonat ausgewählt wird.

11. Die pharmazeutische Brausezusammensetzung gemäß Ansprüche 1-10 und **dadurch gekennzeichnet, dass** die genannte Zusammensetzung 5-50% Ceftibutenoder seine pharmazeutisch annehmbaren Solvaten, Monohydratform, Dihydratform, Trihydratform, Enantiomere, Racemate, organischen Basen, anorganischen Basen, Polymorphe, Kristall und amorphen Formen in Bezug auf das Gesamtgewicht der Einheitsdosis enthalten kann.

12. Die pharmazeutische Brausezusammensetzung gemäß Ansprüche 1-11 und **dadurch gekennzeichnet, dass** die genannte Zusammensetzung 5-50% Ceftibuten; 1-5% Povidon als Bindemittel; 0,1-8% Schmiermittel; 0-15% Sprengmittel und/oder Sprengmittel; 0-55% Verdünnungsmittel; 1-8% Geschmack regulierendes Mittel; 0,5-5% Geschmacksstoffe und 10-45% Brausesäure aus und 20-75% Brausebasis in Bezug auf die Gesamtmenge der Dosiseinheit enthält.

13. Die pharmazeutische Brausezusammensetzung gemäß Ansprüche 1-12 und **dadurch gekennzeichnet, dass** die genannte Zusammensetzung durch ein Verfahren vorbereitet wird, das Trockenmischen oder trockene Granulation oder Feuchtgranulierung der Bestandteile oder eine Kombination davon enthält.

## Revendications

1. Une composition pharmaceutique effervescente comprenant ceftibutène, **caractérisée en ce que**,
• povidone comme liant ayant une taille particulaire de 60 à 150µm est utilisée,
• le rapport entre ceftibutène et povidone est de 7 :1 à 4 :1, et,
• le taux de povidone est de 1 à 5% du poids total de la dose unitaire.

2. Une composition pharmaceutique effervescente selon la revendication 1, dans laquelle ceftibutène peut être sous la forme de ses solvates, monohydratés, dihydratés, trihydratés, esters, énantiomères, racémates, sels organiques, sels inorganiques, polymorphes, sous la forme cristalline et amorphe ou bien sous la forme libre et/ou d'une combinaison de ceux-ci.

3. Une composition pharmaceutique effervescente selon la revendication 2, dans laquelle ceftibutène est sous la forme de dihydraté et/ou trihydraté.

4. Une composition pharmaceutique effervescente selon les revendications 1-3, dans laquelle ladite composition comprend des lubrifiants, désintégrants, diluants, agents régulateurs de goût, un acide effervescent et une base effervescente comme des excipients pharmaceutiquement acceptables.

5. Une composition pharmaceutique effervescente selon la revendication 4, dans laquelle l'agent régulateur de goût est choisi dans un groupe comprenant acésulfame, aspartame, dextrose, fructose, glucose, lactitol, maltitol, sorbitol, saccharine, saccharine de sodium, saccharose, cyclamate de sodium, sucralose, chlorure de sodium, chlorure de potassium, sucrose, xylitol ou combinaisons de ceux-ci.

6. Une composition pharmaceutique effervescente selon les revendications 1-5, dans laquelle l'agent régulateur de goût est utilisé dans un rapport de 1-8% par rapport le poids de la dose unitaire.

7. Une composition pharmaceutique effervescente selon la revendication 6, dans laquelle le rapport entre ceftibutène et l'agent régulateur de goût est de 1:1 à 4:1.

8. Une composition pharmaceutique effervescente selon la revendication 7, dans laquelle le rapport entre ceftibutène et l'agent régulateur de goût est 3:1.

9. Une composition pharmaceutique effervescente selon la revendication 4, dans laquelle l'acide effervescent est choisi parmi les acides organiques tels qu'acide citrique, acide tartrique, acide malique, acide fumarique, acide ascorbique et acide adipique.

10. Une composition pharmaceutique effervescente selon la revendication 4, dans laquelle la base effervescent est choisi parmi les agents basiques tels que carbonate hydrogène de sodium, carbonate de sodium, carbonate de potassium, bicarbonate de potassium, carbonate de sodium glycine, carbonate de lysine, carbonate d'arginine et carbonate de calcium.

11. Une composition pharmaceutique effervescente selon les revendications 1-10, dans laquelle ladite composition peut comprendre ceftibutène ou bien ses solvates pharmaceutiquement acceptables, sa forme monohydraté, dihydraté, trihydraté, énantiomères, racémates, bases organiques, bases inorganiques, polymorphes, sous la forme cristalline et amorphe dans un rapport de 5-50% par rapport au poids total de la dose unitaire.

12. Une composition pharmaceutique effervescente selon les revendications 1-11, dans laquelle ladite composition comprend 5-50% ceftibutène; 1-5% povidone comme liant, 0,1-8% lubrifiant; 0-15% désintégrant ebou désintégrants ; 0-55% diluant ; 1-8% l'agent régulateur de goût ; 0,5-5% aromatisants et 10-45% acide effervescent et 20-75% base effervescente par rapport au poids total de la dose unitaire.

13. Une composition pharmaceutique effervescente selon les revendications 1-12, dans laquelle ladite composition est préparée par la méthode composée de mélange à sec ou de granulation à sec ou de granulation humide des components ou une combinaison de ceux-ci.
